# EUROPÄISCHE PATENTANMELDUNG

(11) **EP 2 422 824 A2**
(43) Veröffentlichungstag der Anmeldung: **29.02.2012**
(21) Anmeldenummer: 11007016.6
(22) Anmeldetag: 29.08.2011
(51) Int. Cl.: A61L 27/16, A61L 27/44, A61L 27/54, A61K 6/083

(54) **Prothesenausgangsmaterial, insbesondere für Dentalprothesen**

(30) Priorität: 30.08.2010 DE 102010035867
(71) Anmelder: Retec Kunststofftechnik GmbH, 61191 Rosbach v.d. Höhe (DE)
(72) Erfinder: Reiss, Siegfried, 61191 Rosbach (DE)
(74) Vertreter: Metten, Karl-Heinz

(57) **Zusammenfassung**

Die vorliegende Erfindung betrifft ein Prothesenausgangsmaterial, enthaltend Silbersulfat. Ferner betrifft die vorliegende Erfindung ein Prothesenausgangsmaterial, insbesondere ein Prothesenausgangsmaterial für Dentalprothesen, die Verwendung dieses Prothesenausgangsmaterials für die Herstellung von Prothesen sowie insbesondere von Dentalprothesen.

## Beschreibung

Die vorliegende Erfindung betrifft ein Prothesenausgangsmaterial, insbesondere ein Prothesenausgangsmaterial für Dentalprothesen, die Verwendung dieses Prothesenausgangsmaterials für die Herstellung von Prothesen sowie insbesondere von Dentalprothesen.

Prothesenmaterialien haben in vielfältiger Hinsicht hohen Anforderungen zu genügen. Sie haben nicht nur extrem bioverträglich zu sein und sich durch eine sehr hohe Passgenauigkeit über einen sehr langen Zeitraum auszuzeichnen, sondern ebenfalls einen adäquaten Schutz gegen Mikroorganismen zu liefern.

Aus dem Dentalbereich ist die antimikrobielle Wirkung des Dentalwerkstoffs Silberamalgam bekannt.

Antimikrobiell wirksame Kunststoffkörper sollen sich gemäß der DE 199 36 059 A1 dadurch auf besonders einfache Weise herstellen lassen, dass man vor dem eigentlichen Formen des Kunststoffkörpers mindestens einen Bestandteil des Vorprodukts mit einem Metallkolloid behandelt. In der WO 01/43788 A2 wird ein antimikrobiell ausgestatteter Formkörper beschrieben, der über eine Polymerbeschichtung verfügt, welche ein Metallkolloid zu enthalten hat. Kunststoffprothesen, die sich durch eine zufrieden stellende antimikrobielle Wirksamkeit auszeichnen sollen, werden gemäß der DE 103 31 324 A1 dadurch erhalten, dass man ein Vorprodukt zunächst mit einem antimikrobiellen Metallkolloid behandelt und anschließend ein lösliches oder schwer lösliches Salz eines antimikrobiellen Metalls zusetzt.

Ein elastischer Formkörper lässt sich gemäß GB 696 912 A dadurch antimikrobiell austatten, dass man diesen mit einer Komplexverbindung des Silbers, welche zwei Monohydroxy-monoaminoalkan-Subsituenten aufweist, behandelt.

Gemäß der EP 1 375 700 A1 wird ein antimikrobiell ausgestatteter Formkörper dadurch erhalten, dass man eine Silberlage auf der Oberfläche des Formkörpers chemisch abscheidet.

Die DE 10 2006 056 284 A1 geht ein auf ein Verfahren zur Herstellung einer antimikrobiell wirkenden wässrigen Dispersion, enthaltend nanoskalige Teilchen, die wenigstens ein antimikrobiell wirkendes Metall oder eine antimikrobiell wirkende Metallverbindung aufweisen, und ein Polymerisations-, Polykondensations- oder Polyadditionsprodukt. Mittels Weiterverarbeitung erhält man ein festes Zwischenprodukt, das sich zu einem Kunststoffgegenstand oder zu einer Kunststoffbeschichtung weiterverarbeiten lassen soll.

Die DE 10 2005 041 005 A1 beschreibt silberhaltige Zusammensetzungen mit biozider Wirkung, welche neben nanopartikulärem Silber mindestens ein Silbersalz, nanoparkituläres Zinkoxyd, Chitosan oder Chitosanderivat aufzuweisen haben. Aus der WO 01/24839 A1 geht eine biokompatible Polymermatrix aus einem Polyacrylamidgerüst und einem nicht-gelierenden Polysaccharid hervor, welche mit Silber ausgestattet sein kann.

In der DE 603 09 383 T2 wird abgestellt auf Dentalmassen, enthaltend Silber-Zink-Glas, wenigstens ein Monomer mit wenigstens einer ethylenisch ungesättigten Gruppe und ein Polymerisationsinitiatorsystem. Die hieraus erhältlichen Silberkeramiken sollen antimikrobiell ausgestattet sein, ohne dass die mechanische Festigkeit der Dentalzusammensetzungen geopfert werden muss. Allerdings sind keine geringen Silberadditivmengen zu verwenden, um einen einigermaßen vertretbaren antimikrobiellen Effekt zu erzielen. Auch kann eine hinreichende Transparenz, Farbneutralität und Lagerstabilität in der Regel nicht mehr eingehalten werden. Diese Eigenschaften sind jedoch bei Dentalprothesen und Otoplastiken von entscheidender Bedeutung.

Die vorangehend geschilderten, aus dem Stand der Technik bekannten antimikrobiellen Zusammensetzungen lassen insbesondere für die Verwendung als Prothesenmaterialien noch stets Wünsche offen, nicht zuletzt bei dem Grad und der Dauer der antimikrobiellen Wirksamkeit.

Die heutigen Prothesenmaterialien, insbesondere auch im Dentalbereich, haben eine Qualität erreicht, die es gestattet, auch so genannte provisorische Prothesen über einen längeren Zeitraum tragen zu können. Diese Entwicklung trägt dem Umstand Rechnung, dass insbesondere im Dentalbereich sich die Form der endgültigen Prothese häufig erst nach einer längeren Wartezeit korrekt ermitteln lässt. Um diese Zeitspanne angemessen zu überbrücken, gewinnen diese so genannten provisorischen Prothesen mehr und mehr an Bedeutung. Provisorische Prothesen werden im Dentalbereich zuweilen schon für eine Zeitdauer von über 12 Monaten getragen. Die auf Kunststoffmaterialien basierenden provisorischen Prothesen dürfen, insbesondere wenn sie über einen längeren Zeitraum getragen werden, nicht Veranlassung zur Bakterienbildung geben und nicht ein Gefahrenherd für potentielle Entzündungen sein. Dass dieses Problem von vorrangiger Bedeutung ist, dokumentieren nicht zuletzt die vielfältigen aus dem Stand der Technik bekannten Versuche, dem Problem beizukommen.

Der vorliegenden Erfindung lag daher die Aufgabe zugrunde, Prothesenausgangsmaterialien für die Herstellung von Prothesen zur Verfügung zu stellen, die auch über einen längeren Zeitraum eine besonders ausgeprägte antimikrobielle Wirkung zeigen.

Demgemäß wurde ein Prothesenausgangsmaterial, enthaltend Silbersulfat und gegebenenfalls gefälltes Silber gefunden.

Dabei kann vorgesehen sein, dass das Prothesenausgangsmaterial ein polymerisierbares, insbesondere kalt-polymerisierbares Prothesenausgangsmaterial darstellt. Solche polymerisierbaren Prothesenausgangsmaterialien umfassen z.B. so genannte Heißpolymerisate auf der Basis von Pulver und Flüssigkeit. Gemäß DIN EN ISO 1567:2000 liegt die Grenze zwischen kalthärtenden und heißhärtenden Systemen bei 65°C.

Besonders bewährt haben sich als erfindungsgemäße Prothesenausgangsmaterialien so genannte Mehrkomponenten-Prothesenausgangsmaterialien, insbesondere Zweikomponenten-Prothesenausgangsmaterialien, umfassend mindestens eine Fest-, insbesondere Pulverkomponente, und mindestens eine Flüssigkomponente. Besonders bevorzugt bei diesen Systemen sind Zweikomponenten-Prothesenausgangsmaterialien auf der Basis von Polymethylmeth-acrylaten (PMMA), und zwar sowohl als Heiß- als auch als Kaltpolymerisat.

Als Mehr- bzw. 2-Komponenten-Prothesenausgangsmaterialal wird im Sinne der vorliegenden Erfindung eine solche Zusammensetzung verstanden, aus der Prothesenmaterialien bzw. Prothesen oder Bestandteile hiervon, beispielsweise Dentalprothesen, provisorische Kronen und Brücken, kieferorthopädische Teile (sogenannte Orthoplastiken) oder Otoplastiken für Hörgeräte. Geeignete Dentalprothesenmaterialien, die aus den erfindungsgemäßen Prothesenausgangsmaterialien zugänglich sind, umfassen insbesondere die Prothesenbasis, welche mit verbesserten Eigenschaften ausgestattet werden kann. Die Prothesenbasis umfasst bei Dentalprothesen solche Bereiche bzw. Bauteile, in die die Zahnprothesen eingearbeitet werden und die letztendlich für die Anbindung an Areale des Gaumens vorgesehen sind.

Das Silbersulfat und/oder das gefällte Silber liegt in den erfmdungsgemäßen Prothesenausgangsmaterialien vorzugsweise in einer durchschnittlichen Partikelgröße im Bereich von > 0,1 µm bis 20 µm, vorzugsweise im Bereich von 1 µm bis 10 µm, vor. Als besonders bevorzugt haben sich solche Prothesenausgangsmaterialien erwiesen, bei denen Silbersulfat und/oder gefälltes Silber mit einer durchschnittlichen Partikelgröße im Bereich von 1,5 bis 45 µm, besonders bevorzugt mit einer durchschnittlichen Partikelgröße im Bereich von 1,5 bis 10 µm sowie insbesondere im Bereich von 2 bis 9 µm, beispielsweise im Bereich von 5 bis 8 µm, vorliegt. Mit solchen Ausführungsformen sind Prothesen zugänglich, die über einen sehr langen Nutzungszeitraum noch stets mit einer sehr hohen antimikrobiellen Wirksamkeit ausgestattet sind. D.h. die antimikrobielle Wirksamkeit verringert sich nicht signifikant durch Auswaschen oder sonstige Migrationsprozesse.

Bezogen auf das Gesamtgewicht verfügen die erfindungsgemäßen Prothesenausgangsmaterialien vorteilhafterweise über eine Menge an gefälltem Silber im Bereich von 0,01 bis 5 Gew.-%, bevorzugt von 0,1 bis 1 Gew.-%, bezogen auf das Gesamtgewicht des Prothesenausgangsmaterials, Gew.-% und/oder über eine Menge von 0,01 bis 5 Gew.%, bevorzugt von 0,1 bis 1 Gew.-%, an Silbersulfat. Zweckmäßige Ausgestaltungen setzen gefälltes Silber und/oder Silbersulfat in Mengen von, mindestens 0,15 oder mindestens 0,2 Gew.-%, bezogen auf das Gesamtgewicht des Prothesenausgangsmaterials, ein.

Besonders bevorzugte erfindungsgemäße Prothesenausgangsmaterialien auf der Basis von Mehrkomponenten-Prothesenausgangsmaterialien umfassen mindestens eine flüssige Komponente (Komponente A), enthaltend mindestens ein Alkylmethacrylat, insbesondere Methylmethacrylat, und mindestens eine Feststoffkomponente (Komponente B), enthaltend mindestens ein Homo- und/oder Copolymer eines Alkyl(methacrylats), insbesondere Methylmethacrylats.

Die Feststoffkomponente (Komponente B) stellt hierbei regelmäßig ein Schüttgut dar, dass vorzugsweise in pulverförmiger oder rieselfähiger Konsistenz vorliegt.

In einer zweckmäßigen Ausgestaltung ist vorgesehen, dass das Homo- und/oder Copolymer des Alkyl(meth)acrylats in Pulverform und/oder in Form von Perlen in der Feststoffkomponente (B) vorliegt. Hierbei ist von Vorteil, wenn die Perlen zumindest teilweise vernetzt vorliegen.

Das Gewichtsverhältnis der Feststoffkomponente (B) zu der Flüssigkomponente (A) liegt vorteilhafterweise im Bereich von 10:8 bis 10:2, vorzugsweise im Bereich von 10:6 bis 10:2 und insbesondere im Bereich von 10:7 bis 10:2,5. Es lassen sich beispielsweise ohne weiteres Gewichtsverhältnisse von 10:4 oder 10:3 einstellen. Indem man auf Mischungen zurückgreifen kann mit einem geringen Anteil an Flüssigkomponente, d.h. des zu polymerisierenden Monomers, resultiert mit den erfindungsgemäßen Prothesenausgangsmaterialien stets ein sehr geringer Restmonomergehalt in den hergestellten Prothesenmaterialien, und zwar sowohl mit Heiß- als auch mit Kaltpolymerisaten.

Perlpolymerisate, insbesondere solche aus (Meth)acrylaten, sind dem Fachmann im Allgemeinen bekannt. Perlpolymerisate auf der Basis von Polyalkyl(meth)acrylaten, insbesondere Polymethylmethacrylaten, vor allem solche mit kleinen durchschnittlichen Partikelgrößen, beispielsweise im Bereich von 1 µm bis 30 µm, lassen sich z. B. mittels Fällungspolymerisation erhalten. Im Wege der Suspensionspolymerisation sind üblicherweise Perlpolymerisate mit durchschnittlichen Teilchengrößen größer als 35 µm ohne weiteres zugänglich. Exemplarisch sei in diesem Zusammenhang auf die Offenbarung der EP 0 443 609 A2 verwiesen, auf welche hiermit ausdrücklich Bezug genommen wird. Gemäß der DE 100 65 501 A1 gelangt man mittels Suspensionspolymerisation zu Perlpolymerisaten mit durchschnittlichen Partikelgrößen im Bereich von 1 µm bis 40 µm, wenn die polymerisierbare Zusammensetzung mindestens 50 Gew.-% (Meth)acrylate aufweist und wenn in wässriger Phase dispergiert und polymerisiert wird. Die Suspension ist mit Hilfe von Aluminiumverbindungen zu stabilisieren. Vernetzte Perlpolymerisate mit einem mittleren Partikeldurchmesser von 10 bis 100 µm auf der Basis von Methacrylatpolymeren entnimmt man DE 28 50 916. Als geeignete multifunktionelle Vernetzermoleküle werden dort Ethylenglycoldimethacrylat und Divinylbenzol explizit genannt.

In einer weiteren zweckmäßigen Ausgestaltung liegt in der Komponente B mindestens ein Vernetzer, insbesondere im Bereich von 0,1 Gew.-% bis 10 Gew.-% oder im Bereich von 0,5 Gew.-% bis 5 Gew.-% als vernetztes Comonomer einpolymerisiert vor. Des weiteren kann die Komponente B eine mit mindestens einer elastischer Phase modifiziertes personifiziertes Perlpolymerisat darstellen.

Für die Vernetzung greift man regelmäßig auf multifunktionelle Comonomere oder auch multifunktionelle Oligomere zurück. Neben di-, tri- und polyfunktionellen (Meth)acrylaten eignen sich hierfür auch Pfropfvernetzer mit mindestens zwei unterschiedlichen reaktiven C-C-Doppelbindungen, beispielsweise Alkylmethacrylaten und Alkylacrylaten, sowie aromatische Vernetzer wie 1,2-Divinylbenzol, 1,3-Divinylbenzol und 1,4-Divinylbenzol. Unter den di-funktionellen (Meth)acrylaten seien insbesondere die (Meth)acrylate des Propandiols, Butandiols, Hexandiols, Octandiols, Nonandiols, Decandiols und Eicosandiols sowie ferner die Di(meth)acrylate des Ethylenglycols, Triethylenglycols, Tetraethylenglycols, Dodecaethylenglycols, Tetradecaethylenglycols, Propylenglycols, Dipropylenglycols und Tetradecapropylenglycols, außerdem Glycerindi(meth)acrylat, 2,2-bis [(gamma-methacryloxy-beta-oxypropoxy)-phenylpropan], bis-GMA, Bisphenol-A-dimethacrylat, Neopentylglycoldi(meth)acrylat, 2,2-dimethacryloxypolyethoxyphenyl)propan mit 2 bis 10 Ethoxygruppen pro Molekül sowie 1,2-Bis(3-methacryloxy-2-hydroxypropoxy)butan genannt. Exemplarisch seien als multifunktionelle (Meth)acrylate, z. B. Di-, Tri- und/oder Tetra(meth)acrylate, wie 1,4-Butandioldimethacrylat, Ethylenglycoldimethacrylat und Urethandimethacrylat, sowie di- oder trivinylische Verbindungen wie Divinylbenzol hervorgehoben. Selbstverständlich können auch Mischungen der genannten Vernetzermoleküle zum Einsatz kommen. Besonders geeignet sind auch solche multifunktionellen Verbindungen, insbesondere di-und/oder tri- funktionelle Verbindungen, die elastische Einheiten besitzen und demgemäß geeignet sind, die aus den Prothesenausgangsmaterialien erhaltenen Prothesenmaterialien mit flexiblen Eigenschaften auszustatten.

Als geeignete Alkylmethacrylate für die flüssige Komponente A sei auf Methyl-, Ethyl-, n- Propyl-, i-Propyl-, n-Butyl, t-Butyl, i-Butyl, Benzyl- und Furfurylmethacrylate oder deren Mischungen verwiesen. Methylmethacrylat ist besonders bevorzugt als Monomer für die Flüssigkomponente (Komponente A).

Das für die Polymerisation erforderliche radikalische Initiatorsystem ist, je nach Reaktionsbedingungen bzw. Polymerisationssystem, in der flüssigen Komponente A und/oder der Feststoffkomponente (Komponente B) enthalten. Diesbezügliche Details sind dem Fachmann bekannt. Beispielsweise liegt bei Basismischungen für Kaltpolymerisate das Initiatorsystem meist in beiden Komponenten, der flüssigen Komponente und der Feststoffkomponente vor und wird demgemäß beim Mischen dieser Komponenten zusammengerührt. Bei den Basismaterialien für Heißpolymerisate liegt der Initiator meist in der Polymerkomponente, d.h. der Feststoffkomponente vor. Erst beim Mischen gelangt der Initiator dann in die flüssige Monomerkomponente. Demgemäß können Prothesenausgangsmaterialien vorgesehen sein, bei denen in der Feststoffkomponente (B) ein Gehalt an reaktiver Initiatorkomponente, insbesondere in Form von Peroxiden, Perketalen, Perestern, Azoverbindungen und/oder Barbitursäure, Thiobarbitursäure, Barbitursäurederivaten bzw. Thiobarbitursäurederivaten, vorliegt. Hierbei kann es sich z.B. auch um Restgehalte an bei der Herstellung der Feststoffkomponenten nicht abreagierter Initiatorkomponente handeln, z.B. um Peroxide wie Dibenzoylperoxid.

Als Initiatoren für die Polymerisationsreaktion von Kalt- wie auch Heißpolymerisat-Ausgangsmischungen kommen grundsätzlich solche in Betracht, mit denen sich radikalische Polymerisationsreaktionen starten lassen. Bevorzugte Initiatoren sind Peroxide wie Dibenzoylperoxid, Dilauroylperoxid, t-Butanperbenzoat und Di-t-butyl-peroxid sowie Azoverbindungen wie Azobis(isobutyronitril) (AIBN) oder Azobis(4-cyanvaleriansäure). Für die Heißhärtung kann des Weiteren auch auf Perketale und Benzpinakole zurückgegriffen werden.

Um die Initiierung der radikalischen Polymerisation durch Peroxide zu beschleunigen, können geeignete Aktivatoren, z.B. aromatische Amine, hinzugefügt werden. Exemplarisch seien als geeignete Amine N,N-Dimethyl-p-toluidin, N,N-Dihydroxyethyl-p-toluidin und p- Dibenzylaminobenzoesäurediethylester genannt. Hierbei fungieren die Amine regelmäßig als Co-Initiatoren und liegen üblicherweise in einer Menge von bis zu 3 Gew.- % vor.

Als radikalische Initiatorsysteme sind weiterhin Redoxsysteme geeignet, insbesondere Kombinationen aus Dibenzoylperoxid, Dilauroyl oder Campherchinon mit Aminen wie N, N-Dimethyl-p-toluidin, N-N-Dihydroxyethyl-p-toluidin und p-Dimethylaminobenzoesäure-diethylester. Ferner können als Redoxsysteme auch solche zum Einsatz kommen, die neben einem Peroxid auch noch Ascorbinsäure oder deren Derivate, Barbitursäure oder ein Barbitursäurederivat oder eine Sulfinsäure als Reduktionsmittel enthalten. Besonders geeignete Barbitursäurederivate umfassen in 5-Position substituierte Barbitursäure, z.B. 1-Benzyl-5-Phenyl-barbitursäure, 5-n-Butylbarbitursäure und S-n-Cyclohexyl-barbitursäure. In einer zweckmäßigen Ausführungsform enthält ein solches Redoxsystem Barbitursäure oder Thiobarbitursäure oder ein Barbitursäure- oder Thiobarbitursäurederivat (beispielsweise 0,5 *-* 5% Gew.- %), mindestens eine peroxidische Verbindung, z.B. eine Peroxodisulphat- und/oder Peroxodiphosphatverbindung (beispielsweise 25 bis 65 Gew.- %), mindestens ein Kupfersalz oder ein Kupferkomplex (beispielsweise 0,01 bis 8 Gew.- %, vorzugsweise 0,01 - 1 Gew.-%) und mindestens eine Verbindung mit einem ionogen vorliegenden Halogenatom (beispielsweise 0,05 bis 7 Gew.- %, vorzugsweise 0,05 - 1 Gew.-%). Exemplarisch seien als geeignete Bestandteile des vorangehend genannten Redoxsystems in 5-Position substituierte Barbitursäure wie 1-Benzyl-5-Phenyl-barbitursäure, 5-n-Butylbarbitursäure, 5-n-Cyclohexyl-barbitursäure, Kupfernaphthenat, Kupfer(II)chlorid-dihydrat, Dilauryldimethylammoniumchlorid, Trioctylmethylammoniumchlorid, Natriumperoxodisulphat, Kupferacetylacetonat und/oder Benzyldibutylammoniumchlorid genannt. Alternativ können auch Photoinitiatoren, welche mittels UV-oder mittels sichtbaren Lichts gestartet werden, zum Einsatz kommen. Geeignete Photoinitiatoren sind z.B. Dialkylbenzilketone, Bisacylphosphinoxide, alpha-Diketone, Phoril, Anisil oder 4,4-Dichlorbenzil oder deren Mischungen.

Insbesondere für kalthärtende polymerisierbare Prothesenausgangsmaterialien hat es sich als vorteilhaft erwiesen, auf Redoxsysteme zurückzugreifen, die auf Barbitursäure, Thiobarbitursäure, Barbitursäurederivaten und/oder Thiobarbitursäurederivaten basieren oder diese enthalten. Auf diese Weise lässt sich regelmäßig eine gesteigerte Lagerstabilität erzielen. Insbesondere bei Verwendung solcher Prothesenausgangsmaterialien wird, anders als bei manchen kalthärtenden Amin/Peroxidsystemen, bei Einsatz von Silber und/oder Silbersulfat das Problem der Verfärbung in den Prothesenausgangsmaterialien sowie in den daraus erhältlichen Prothesen nicht beobachtet.

Ferner kann man die Feststoffkomponente (B) und/oder die Flüssigkomponente (A) in bekannter Weise, je nach den Polymerisationsbedingungen (Kalt-/Heißpolymerisation), mit weiteren Zusatzstoffen versehen, beispielsweise mit mindestens einem Stabilisator, mindestens einem UV-Absorber, mindestens einem Thixotropiermittel, mindestens einem Füllstoff oder Mischungen der vorangehenden Komponenten.

Als Füllstoffe kommen z.B. in Betracht pyrogene oder Fällungskieselsäuren, Dentalgläser wie Aluminosilikatgläser oder Fluoroaluminosilikatgläser, Strontiumsilikat, Strontiumborsilikat, Schichtsilikate, Zeolithe, Metalloxide oder Fasern oder deren Mischungen. Geeignete Fasern umfassen z.B. Glasfasern sowie Polyamid- oder Kohlenstofffasern.

Erfindungsgemäße Prothesenausgangsmaterialien zeichnen sich in einer Ausgestaltung dadurch aus, dass die Flüssigkomponente (A) mindestens eine zur Vernetzung befähigte Vernetzer-Comonomerkomponente und/oder mindestens einen Polymerisationsbeschleuniger enthält und/oder dass die Feststoffkomponente (B) mindestens einen Initiator enthält.

In den erfindungsgemäßen Prothesenausgangsmaterialien und/oder in den aus diesen Prothesenausgangsmaterialien erhältlichen erfindungsgemäßen Prothesenmaterialien liegen das gefällte Silber und/oder das Silbersulfat vorzugsweise im wesentlichen homogen verteilt vor.

Die der Erfindung zugrunde liegende Aufgabe wird des weiteren gelöst durch ein Prothesenmaterial, insbesondere durch Dentalprothesen, erhältlich aus dem erfindungsgemäßen Prothesenausgangsmaterial.

Der vorliegenden Erfindung liegt die überraschende Erkenntnis zugrunde, dass sich mit Silbersulfat Prothesenmaterialien, insbesondere Dentalprothesen, sehr wirksam antimikrobiell ausstatten lassen, und zwar insbesondere dann, wenn auf Silbersulfatpartikel zurückgegriffen wird, die eine durchschnittliche Partikelgröße oberhalb von 0,1 µm besitzen. Ferner wurde gefunden, dass bei den erfindungsgemäßen Prothesenausgangsmaterialien sowie den daraus erhältlichen Prothesenmaterialien das darin vorliegende Silbersulfat und das gegebenenfalls darin enthaltene gefällte Silber nicht dazu tendieren, aus der Kunststoffmatrix mittels Migration herauszugelangen, und zwar insbesondere wenn die durchschnittliche Partikelgröße des Silbersulfats und/oder des gefällten Silbers größer 0,1 µm ist. Die erfindungsgemäßen Prothesenausgangsmaterialien sowie die daraus erhältlichen erfindungsgemäßen Prothesenmaterialien zeigen auch im Dauereinsatz noch stets eine hohe antimikrobielle Wirkung. Des Weiteren wurde bei den erfindungsgemäßen Prothesenausgangsmaterialien und den daraus erhältlichen erfindungsgemäßen Prothesenmaterialien selbst bei Langzeitstudien keine bzw. nur eine sehr vemachlässigbare Verfärbung festgestellt. Die erfindungsgemäßen Produkte zeichnen sich zudem durch eine gute und verlässliche Transparenz und Farbneutralität aus.

Zur besseren Veranschaulichung wird die vorliegende Erfindung anhand der nachfolgenden Beispiele im Detail erläutert. Selbstverständlich ist die vorliegende Erfindung nicht auf diese Beispiele beschränkt.

Beispiele:
Prüfkörper wurden über ein Zweikomponenten-Prothesenausgangsmaterial auf PMMA-Basis erhalten (retec press LT). Die erhaltenen Prüfkörper wurden gemäß dem Teststandard JIS Z 2801 auf ihre antimikrobielle Wirkung untersucht. Es wurde eine Prüfsuspension des folgenden Untersuchungsorganismus eingesetzt: *Escherichia coli* ATTC 8739 (1,0 x 10⁶ KBE/Testfläche) (KBE = koloniebildende Einheiten).

Die Verringerung der antimikrobiellen Wirksamkeit wurde wie folgt bestimmt: Reduktion = log(unbeh. Probe 0h / unbeh. Probe 24h) - log(behandelte Probe 24h / unbeh. Probe 0h). Die Reduktion wird in den nachfolgenden Tabellen in log-Stufen angegeben.

Der vorgenommenen Beurteilung lag das folgende Beurteilungsschema zugrunde:
Reduktion nach 24 h < 1 Zehnerpotenz =̂ keine signifikante bakterizide/fungizide Wirkung
Reduktion nach 24 h ≥ 1 Zehnerpotenz < 2 Zehnerpotenzen =̂ geringe bakterizide/fungizide Wirkung
Reduktion nach 24 h ≥ 2 Zehnerpotenzen < 3 Zehnerpotenzen signifikante bakterizide/fungizide Wirkung
Reduktion nach 24 h ≥ 3 Zehnerpotenzen =̂ starke bakterizide/fungizide Wirkung

Für Lagezeiten von 24h, 8 Wochen und 12 Monate wurden die folgenden Resultate erhalten:
Tabelle 1: Lagerzeit der Prüfkörper: 24h bei 37°C in demineralisiertem
   Wasser

| | KBE*/Testfläche Nach 24h | |
|---|---|---|
| Prüfkörper - 0 % Silbersulfat | 9,3 x 10⁶ | (nach 0h 4,8x10⁵) |
| Prüfkörper- 1,0% Silbersulfat | <10 | Reduktion > 6,0 |
| Prüfkörper - 0,5% Silbersulfat | <10 | Reduktion > 6,0 |
| Prüfkörper-0,1% Silbersulfat | <10 | Reduktion > 6,0 |
| Prüfkörper - 0,1% Silbersulfat + 0, 1 % Silber gefällt | <10 | Reduktion > 6,0 |

| | | |
|---|---|---|
| *KBE = Koloniebildende Einheiten | | |

**Tabelle 2: Lagerzeit der Prüfkörper: 8 Wochen bei 37°C in demineralisiertem Wasser**

| | KBE*/Testfläche Nach 24h | |
|---|---|---|
| Prüfkörper - 0 % Silbersulfat | 9,8 x 10⁶ | (nach 0h 3,4x10⁵) |
| Prüfkörper- 1,0 % Silbersulfat | < 10 | Reduktion > 5,9 |
| Prüfkörper - 0,5% Silbersulfat | < 10 | Reduktion > 5,9 |
| Prüfkörper - 0,1 % Silbersulfat | < 10 | Reduktion > 5,9 |

| | | |
|---|---|---|
| *KBE = Koloniebildende Einheiten | | |

**Tabelle 3: Lagerzeit der Prüfkörper: 12 Monate bei 37°C in demineralisiertem Wasser**

| | KBE* 1/Testfläche Nach 24h | |
|---|---|---|
| Prüfkörper - 0 % Silbersulfat | 8,0 x 10⁶ | (nach 0h 5,4x10⁵) |
| Prüfkörper- 1,0 % Silbersulfat | < 10 | Reduktion > 6,0 |
| Prüfkörper - 0,5 % Silbersulfat | < 10 | Reduktion > 6,0 |
| Prüfkörper - 0,1 % Silbersulfat | 5,7 x 10⁶ | Reduktion 0,1 |
| Prüfkörper - 0,1 % Silber (gefällt)¹⁾ | 1,1 x 10⁷ | Reduktion 0,0 |

| | | |
|---|---|---|
| *¹ KBE = Koloniebildende Einheiten ¹⁾ Der Testkörper wurde nur 9 Monate gelagert. | | |

Die in der vorstehenden Beschreibung und in den Ansprüchen offenbarten Merkmale der Erfindung kann sowohl einzeln als auch in jeder beliebigen Kombination für die Verwirklichung der Erfindung in ihren verschiedenen Ausführungsformen wesentlich sein.

## Patentansprüche

1. Prothesenausgangsmaterial, enthaltend Silbersulfat.

2. Prothesenausgangsmaterial nach Anspruch 1, ferner enthaltend gefälltes Silber.

3. Prothesenausgangsmaterial nach Anspruch 1 oder 2, **dadurch gekennzeichnet, dass** dieses ein polymerisierbares, insbesondere kalt-polymerisierbares, Prothesenausgangsmaterial darstellt.

4. Prothesenausgangsmaterial nach einem der vorangehenden Ansprüche, **dadurch gekennzeichnet, dass** dieses ein Mehrkomponenten-Prothesenausgangsmaterial, insbesondere Zweikomponenten-Prothesenausgangsmaterial, darstellt, umfassend mindestens eine Fest-, insbesondere Pulverkomponente, und mindestens eine Flüssigkomponente.

5. Prothesenausgangsmaterial nach einem der vorangehenden Ansprüche, **dadurch gekennzeichnet, dass** das Silbersulfat und/oder das gefällte Silber eine durchschnittliche Partikelgröße im Bereich größer 0,1 µm bis 20 µm, vorzugsweise im Bereich von 1 µm bis 10 µm, aufweist.

6. Prothesenausgangsmaterial nach einem der Ansprüche 2 bis 5, **dadurch gekennzeichnet, dass** das gefällte Silber in einer Menge im Bereich von 0,01 bis 5 Gew.-%, bevorzugt von 0,1 bis 1 Gew.-%, bezogen auf das Gesamtgewicht des Prothesenausgangsmaterials, in diesem vorliegt und/oder
dass das Silbersulfat in einer Menge im Bereich von 0,01 bis 5 Gew.-%, bevorzugt von 0,1 bis 1 Gew.-%, bezogen auf das Gesamtgewicht des Prothesenausgangsmaterials, in diesem vorliegt.

7. Prothesenausgangsmaterial nach einem der Ansprüche 4 bis 6, **dadurch gekennzeichnet, dass** mindestens eine flüssige Komponente (Komponente A) mindestens ein Alkylmethacrylat, insbesondere Methylmethacrylat, und mindestens eine Feststoffkomponente (Komponente B), mindestens ein Homo- und/oder Copolymer eines Alkyl(methacrylats), insbesondere des Methylmethacrylats, enthalten, wobei das Homo- und/oder Copolymer des Alkyl(meth)acrylats vorzugsweise in Pulverform und/oder in Form von Perlen vorliegt.

8. Prothesenausgangsmaterial nach Anspruch 7, **dadurch gekennzeichnet, dass** die Perlen des Copolymerisats zumindest teilweise vernetzt vorliegen.

9. Prothesenausgangsmaterial nach Anspruch 7 oder 8, **dadurch gekennzeichnet, dass** das Gewichtsverhältnis der Komponente B zu der Komponente A im Bereich von 10:8 bis 10:2, insbesondere im Bereich von 10:7 bis 10:2,5, liegt.

10. Prothesenausgangsmaterial nach einem der Ansprüche 7 bis 9, **dadurch gekennzeichnet, dass** in der Komponente B mindestens ein Vernetzer, insbesondere im Bereich von 0,1 Gew.-% bis 10 Gew.-% oder im Bereich von 0,5 Gew.-% bis 5 Gew.-%, als vernetztes Comonomer einpolymerisiert vorliegt und/oder dass die Komponente B ein mit mindestens einer elastischen Phase modifiziertes Perlpolymerisat darstellt.

11. Prothesenausgangsmaterial nach einem der Ansprüche 4 bis 10, **dadurch gekennzeichnet, dass** die Flüssigkomponente mindestens eine zur Vernetzung befähigte Vernetzer-Comonomerkomponente und/oder mindestens einen Polymerisationsbeschleuniger enthält und/oder dass die Feststoffkomponente mindestens einen Initiator enthält und/oder
dass die Flüssigkomponente und/oder die Feststoffkomponente ferner mindestens einen Stabilisator, mindestens einen UV-Absorber, mindestens ein Thixotropiermittel, mindestens einen Füllstoff oder Mischungen der vorangehend genannten Komponenten enthalten und/oder dass in der Feststoffkomponente ein Gehalt an reaktiver Initiatorkomponente, insbesondere in Form von Peroxiden, Perketalen, Perestern und/oder Azoverbindungen, vorliegt und/oder
dass das gefällte Silber und/oder das Silbersulfat in der Fest-, insbesondere Pulverkomponente, insbesondere homogen vermengt, vorliegt.

12. Prothesenausgangsmaterial nach einem der vorangehenden Ansprüche, enthaltend, insbesondere in der Feststoffkomponente, mindestens ein Barbitursäurederivat, vorzugsweise in 5-Position substituierte Barbitursäure, besonders bevorzugt 1-Benzyl-5-Phenyl-barbitursäure, 5-n-Butylbarbitursäure und/oder 5-n-Cyclohexyl-barbitursäure.

13. Prothesenausgangsmaterial nach einem der vorangehenden Ansprüche, **dadurch gekennzeichnet, dass** das gefällte Silber und/oder das Silbersulfat darin im Wesentlichen homogen verteilt vorliegen.

14. Prothesenmaterial, insbesondere Dentalprothesen, hergestellt aus dem Prothesenausgangsmaterial gemäß einem der vorangehenden Ansprüche.

15. Prothesenmaterial nach Anspruch 14, **dadurch gekennzeichnet, dass** dieses ein Prothesenbasismaterial, insbesondere für Dentalprothesen, darstellt, in dem das gefällte Silber und/oder das Silbersulfat vorzugsweise im Wesentlichen homogen verteilt vorliegen.

16. Verwendung eines Prothesenausgangsmaterials gemäß einem der Ansprüche 1 bis 13 zur Herstellung von Prothesenmaterial, insbesondere von Dentalprothesen oder Otoplastiken.
